# EUROPEAN PATENT APPLICATION

(11) **EP 3 586 760 A1**
(43) Date of publication of application: **01.01.2020**
(21) Application number: 18179723.4
(22) Date of filing: 26.06.2018
(51) Int. Cl.: A61B 8/08, A61B 8/00

(54) **METHOD AND APPARATUS FOR POSITIONING OF A DOPPLER ULTRASOUND PROBE OF A FETAL MONITOR**

(71) Applicant: Koninklijke Philips N.V., 5656 AE Eindhoven (NL)
(72) Inventor: JIN, Sheng Kim, 5656 AE Eindhoven (NL); ZHANG, Ying Wendy, 5656 AE Eindhoven (NL); YIN, Bin, 5656 AE Eindhoven (NL)
(74) Representative: de Haan, Poul Erik

(57) **Abstract**

A probe placement assistance method for fetal monitoring includes monitoring signal strength versus time (30) of a Doppler ultrasound signal (18) measured by a Doppler ultrasound probe (10). During the monitoring, human-perceptible guidance is presented to move the Doppler ultrasound probe along a trajectory. A peak in the signal strength versus time is detected which exceeds a threshold (T1, T2). Human-perceptible guidance is then presented to stop movement of the Doppler ultrasound probe. The signal strength versus time may be a signal strength value of the Doppler ultrasound signal for each time bin of a time sequence of time bins. The guidance may be repeated for different, e.g. orthogonal, trajectories until a peak exceeding a second threshold (T2) is reached, after which fetal monitoring begins.

## Description

### FIELD OF THE INVENTION

The following relates generally to the fetal monitoring arts, Doppler ultrasound arts, home fetal monitoring arts, and related arts.

### BACKGROUND OF THE INVENTION

Doppler ultrasound is a common modality for fetal monitoring. In particular, Doppler ultrasound can be used to detect and monitor the fetal heartbeat and fetal movements. Via an ultrasound probe, a fetal monitor acquires fetal heart movement signals and calculates fetal heart related parameters to assist medical professionals in monitoring and diagnosing fetal health status. Increasingly, Doppler ultrasound is being employed in home settings as well, with a parent or other layperson being expected to position the ultrasound probe and acquire Doppler ultrasound measurements of the fetus without professional medical assistance.

However, proper use of Doppler ultrasound monitoring for fetal monitoring requires precise placement of the ultrasound probe in the optimal position for detecting the fetal heartbeat. Medical professionals such as sonographers or ultrasound technicians rely upon their experience to properly position the probe, relying upon audible feedback generated by the Doppler ultrasound device. However, this approach may be difficult for a lay user in a home setting. Additionally, in some settings it may be desirable to have the audio output of the Doppler ultrasound device turned off, e.g. in the case of a sonographer or ultrasound technician performing measurements on a sleeping mother who might be disturbed by the audio output.

One approach for addressing these problems is to employ an ultrasound probe array, i.e. a two-dimensional array of ultrasound probes secured together as a unit. The signals from each probe can be measured, and the probe with the strongest signal is used. However, the ultrasound probe array substantially increases equipment cost.

Pandit, U.S. Pub. No. 2009/0204002 A1, discloses an approach for silently repositioning an ultrasound probe in a case where the fetus or probe has moved so as to degrade or lose the fetal heartbeat signal. This approach leverages the audio channel of the Doppler ultrasound device by not driving the loudspeaker but rather displaying a signal strength indicator on the display of the fetal monitor, e.g. as a bar graphic in which the number of bars shown indicates signal strength. The signal strength indicator is used by the caregiver as a silent visual feedback indicator for positioning of the transducer at a location of strong signal strength. The signal strength indicator is first calibrated by monitoring the audio channel input signal to the transducer to determine the average maximum strength of the audio channel input signal and the average minimum strength of the audio channel input signal. Full scale for the signal strength indicator is then set based on these average maximum and minimum signal strength values. This approach appears to be only useful for repositioning of the ultrasound probe, since the probe must be initially positioned to provide a strong signal in order to perform the signal strength indicator calibration. Thus, the problem of initial positioning of the ultrasound probe remains. Moreover, there is no assurance that the manual scanning of the ultrasound probe will be effective to detect the optimal position for detecting the fetal heartbeat. Still further, the initial signal strength indicator calibration may become inaccurate if, for example, the fetus moves in a way that reduces the signal strength at the optimal position for detecting the fetal heartbeat.

The following discloses certain improvements.

### SUMMARY OF THE INVENTION

In some embodiments disclosed herein, a probe placement assistance method for fetal monitoring is disclosed. The method includes monitoring signal strength versus time of a Doppler ultrasound signal measured by a Doppler ultrasound probe. During the monitoring, human-perceptible guidance is presented to move the Doppler ultrasound probe along a trajectory. Then, a peak in the signal strength versus time is detected which exceeds a threshold. Then, human-perceptible guidance is presented to stop movement of the Doppler ultrasound probe.

In some embodiments disclosed herein, a fetal monitoring device is disclosed, comprising: a fetal monitor; a Doppler ultrasound probe operatively connected with the fetal monitor; one or more electronic processors each integral with or operatively connected with the fetal monitor; and a non-transitory storage medium storing instructions which are readable and executable by the one or more electronic processors to perform a fetal monitoring method including monitoring signal strength versus time of a Doppler ultrasound signal measured by the Doppler ultrasound probe and, during the monitoring, performing at least one iteration of a probe placement assistance method. The probe placement assistance method includes presenting human-perceptible guidance to move the Doppler ultrasound probe along a trajectory and then detecting a peak in the signal strength versus time which exceeds a threshold and then presenting human-perceptible guidance to stop movement of the Doppler ultrasound probe. After completion of a last iteration of the probe placement assistance method, fetal monitoring is performed using the Doppler ultrasound probe including at least monitoring a fetal heart rate.

In some embodiments disclosed herein, a non-transitory computer readable medium has stored thereon program code readable and executable by one or more electronic processors to perform operations including: monitoring signal strength versus time of a Doppler ultrasound signal measured by a Doppler ultrasound probe; and, during the monitoring, performing a probe placement assistance method including presenting human-perceptible guidance to move the Doppler ultrasound probe along a trajectory and then detecting a peak in the signal strength versus time which exceeds a threshold and then presenting human-perceptible guidance to stop movement of the Doppler ultrasound probe.

One advantage resides in providing an approach for positioning the ultrasound probe of a fetal monitor employing Doppler ultrasound monitoring which is readily performed by a lay person without professional medical training or experience.

Another advantage resides in providing such an approach that does not require a calibration process prior to its use.

Another advantage resides in providing such an approach which guides the user during the probe positioning process to ensure that the ultrasound probe is optimally positioned for detecting the fetal heartbeat.

Another advantage resides in providing such an approach that is particularly tuned to the fetal heartbeat signal, so as to minimize interference from the mother's heartbeat, fetal movements, or other extraneous signal sources.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The invention may take form in various components and arrangements of components, and in various steps and arrangements of steps. The drawings are only for purposes of illustrating the preferred embodiments and are not to be construed as limiting the invention.
Fig. 1 diagrammatically illustrates a fetal monitor and a mobile device wirelessly linked with the fetal monitor and running a fetal monitor application ("app") that provides guidance for ultrasound probe placement.
Fig. 2 shows abdominal diagrams depicting likely good locations for placing the ultrasound probe for three different gestational age ranges.
Figs. 3 and 4 show illustrative user dialogs suitably shown on the mobile device display for guiding initial placement of the ultrasound probe.
Figs. 5 and 6 show illustrative user dialogs suitably shown on the mobile device display for guiding a sweep of the ultrasound probe in a horizontal direction performed as part of the ultrasound probe placement.
Figs. 7 and 8 show illustrative user dialogs suitably shown on the mobile device display for guiding a sweep of the ultrasound probe in a vertical direction performed as part of the ultrasound probe placement.
Fig. 9 diagrammatically shows possible movements to perform ultrasound probe placement that may be performed under guidance of the fetal monitor app of Fig. 1.
Fig. 10 shows an illustrative user dialog suitably shown on the mobile device display indicating successful ultrasound probe placement and commencing fetal monitoring.
Fig. 11 shows an illustrative user dialog suitably shown on the mobile device display indicating unsuccessful ultrasound probe placement and guiding a new initial placement of the ultrasound probe.
Fig. 12 diagrammatically illustrates a more detailed ultrasound probe placement guidance method suitably performed by the mobile device of Fig. 1 running the fetal monitor app that provides guidance for ultrasound probe placement.

### DETAILED DESCRIPTION OF EMBODIMENTS

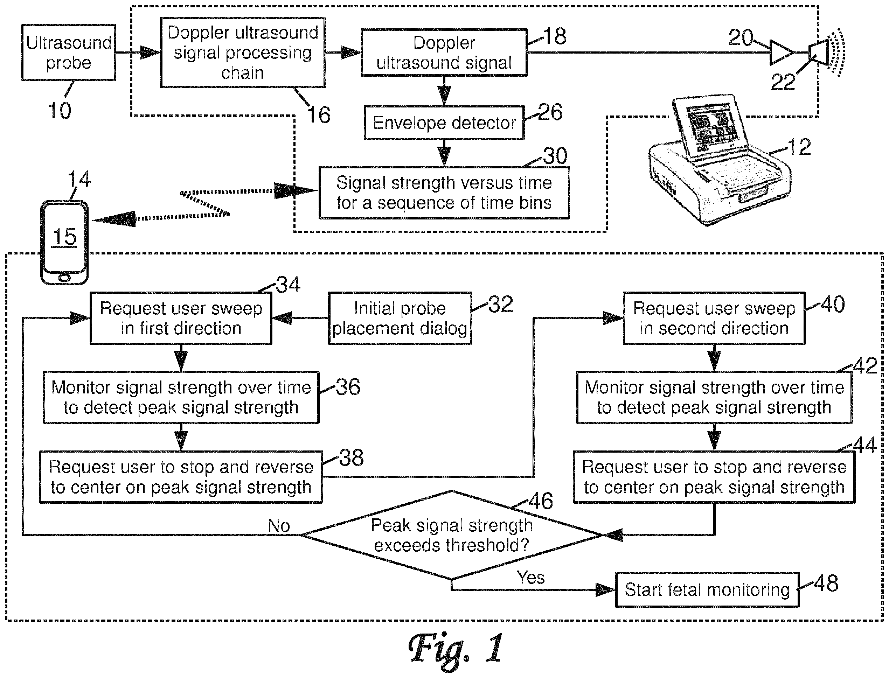

With reference to Fig. 1, an illustrative fetal monitoring device includes a Doppler ultrasound probe 10 that is operatively connected with a fetal monitor 12, and a mobile device (e.g. cellular telephone or tablet computer) 14 with a display 15. The mobile device 14 is wirelessly linked with the fetal monitor 12 (e.g. by a Bluetooth link, Wi-Fi link, or so forth). When used in conjunction with fetal monitoring, the mobile device 14 runs a fetal monitor application ("app"). For example, the fetal monitoring app may be running under a mobile operating system such as iOS or Android. The fetal monitoring app provides guidance for placement of the Doppler ultrasound probe 10 preparatory to performing fetal monitoring, and the fetal monitoring app may also optionally be used to provide data readout during fetal monitoring or so forth. The operative connection of the Doppler ultrasound probe 10 with the fetal monitor 12 may take various forms, such as: (i) a wired connection, i.e. a physical cable connecting the probe 10 with the fetal monitor 12; (ii) a wireless connection, e.g. Bluetooth; or (iii) the operative connection may be by way of the fetal monitor functionality being integrated with the Doppler ultrasound probe 10 as a single physical unit. This latter integrated configuration is facilitated if the fetal monitor app running on the mobile device 14 provides a user interface for receiving user inputs and for displaying fetal heart rate or other clinical data generated by the fetal monitor 12.

Although the illustrative embodiment employs the illustrative mobile device 14, in other embodiments the mobile device 14 may be omitted and user interfacing may be built into the fetal monitor 12, e.g. the fetal monitor 12 may include a display for displaying fetal heart rate or other clinical data. In embodiments (not shown) which omit the mobile device 14, the application providing guidance for placement of the Doppler ultrasound probe 10 is executed by an electronic processor of the fetal monitor 12. These are also not mutually exclusive, e.g. the fetal monitor may include a display and/or other user interfacing components and also be capable of linking with a mobile device.

The foregoing are examples; more generally, one or more electronic processors (e.g. a processor of the fetal monitor 12 and/or the mobile device 14) read and execute instructions stored on a non-transitory computer readable medium (i.e. non-transitory storage medium) to perform operations including monitoring signal strength versus time of a Doppler ultrasound signal measured by a Doppler ultrasound probe 10 and, during the monitoring, performing a probe placement assistance method including presenting human-perceptible guidance to move the Doppler ultrasound probe along a trajectory and then detecting a peak in the signal strength versus time which exceeds a threshold and then presenting human-perceptible guidance to stop movement of the Doppler ultrasound probe. The non-transitory computer readable medium (i.e. non-transitory storage medium) may, by way of non-limiting example, comprise one or more of: a hard disk drive or other magnetic storage medium; a solid state drive (SSD), flash memory, read-only memory (ROM), erasable programmable ROM (EPROM), or other electronic storage medium; an optical disk or other optical storage medium; various combinations thereof, and/or so forth. By way of a specific example, in the illustrative example of Fig. 1 the fetal monitor 12 typically includes an electronic processor and a SSD or other on-board computer readable medium, and the mobile device 14 typically includes an electronic processor and on-board flash memory and/or an SD card or the like.

In operation, an ultrasound transducer or transducer array of the ultrasound probe 10 sonicates the womb at ultrasonic frequency (typically in the megahertz range), and the reflected Doppler ultrasound signal is detected by the same or a different transducer or transducer array of the ultrasound probe 10. In the illustrative embodiment, a signal processing chain 16 of the fetal monitor 12 operate to demodulate the Doppler ultrasound signal to an intermediate frequency (IF; typically in the audio frequency range), and the demodulated Doppler ultrasound signal is digitized by sample-and-hold analog-to-digital (A/D) circuitry to generate the Doppler ultrasound signal 18 that is analyzed by the fetal monitor. The signal processing chain 16 may include variant and/or other operations such as quadrature IQ demodulation, harmonic and/or sideband filtering, and/or so forth, and the processing may be variously partitioned between the analog or digital domains. The signal processing chain 16 may comprise hardware such as, by way of non-limiting illustrative example: analog and/or digital filters; analog and/or digital mixers for performing signal demodulation; a digital signal processing (DSP) processor; field programmable gate array (FPGA); A/D circuitry; various combinations thereof; and/or so forth. Some portion(s) of the signal processing chain 16 may reside in or with (e.g. be integral with) the ultrasound probe 10, and moreover as previously mentioned it is contemplated for the ultrasound probe 10 and fetal monitor 12 to be a single unit.

The illustrative fetal monitor 12 is configured to provide an audible output representing the Doppler ultrasound signal 18. In the illustrative example of Fig. 1, this is achieved by way of a buffered audio amplifier 20 that receives the Doppler ultrasound signal 18 and drives a loudspeaker 22 with an amplified version of the Doppler ultrasound signal 18. If the IF frequency is in the audible range then this arrangement produces an audible Doppler ultrasound output. The audio signal processing chain may include other components besides the illustrative audio amplifier 20, e.g. filters to remove undesired high and/or low frequency components, smoothing filters, and/or so forth. The audio components 20, 22 are optional and, if provided, preferably can be switched off if audible output is not desired.

With continuing reference to Fig. 1, in the following, approaches for providing assistance for placing the ultrasound probe 10 in good position for effectively measuring the fetal heartbeat are described. The approaches entail monitoring signal strength versus time of the Doppler ultrasound signal 18 measured by the Doppler ultrasound probe 10. In the illustrative example, an envelope detector 26 generates the signal strength versus time 30. The illustrative envelope detector 26 may be implemented as a rectifier that rectifies the Doppler ultrasound signal 18 and a filter that performs low-pass filtering of the rectified Doppler ultrasound signal. The signal strength versus time 30 may be recorded for a time sequence of time bins, e.g. proportional to a sum of squared samples of the envelope of the Doppler ultrasound signal 18 over the time bin (advantageously corresponding to an energy or power metric), or so forth. The signal strength over a given time bin may be otherwise metered, e.g. as proportional to a maximum value of the Doppler ultrasound signal 18 over the time bin. (The constant of proportionality in these examples may be unity, i.e. "equal to", or may account for the number of data points N, e.g. constant of proportionality 1/N, or may be some other constant). Thus, in such an approach the signal strength versus time 30 is the signal strength value of the Doppler ultrasound signal 18 for each time bin of the time sequence of time bins. For the disclosed probe placement assistance approaches, the goal is to monitor the signal strength versus time 30 of the Doppler ultrasound signal 18 measured by the Doppler ultrasound probe 10 with sufficient time resolution to enable tracking the signal strength of the Doppler ultrasound signal 18 as the ultrasound probe 10 is moved manually (i.e. by hand) at a reasonably slow speed across the abdomen of the mother proximate to the womb. The signal of interest is a fetal heartbeat, which typically has a fetal heart rate on the order of 120 beats/min (i.e. 120 bpm). Hence, a bin size of around 500 milliseconds is expected to provide suitable temporal resolution so that each bin contains at least one fetal heartbeat, although longer or shorter bin size is contemplated.

In the illustrative arrangement of Fig. 1, the signal strength versus time 30 is transmitted to the mobile device 14 which performs a probe placement assistance method (e.g. implemented as a component of the fetal monitoring app running on the mobile device 14) including presenting human-perceptible guidance to move the Doppler ultrasound probe 10 along a trajectory and then detecting a peak in the signal strength versus time 30 which exceeds a threshold and then presenting human-perceptible guidance to stop movement of the Doppler ultrasound probe 10. In other embodiments, the Doppler ultrasound signal 18 is transmitted to the mobile device and the signal strength versus time 30 is computed at the mobile device.

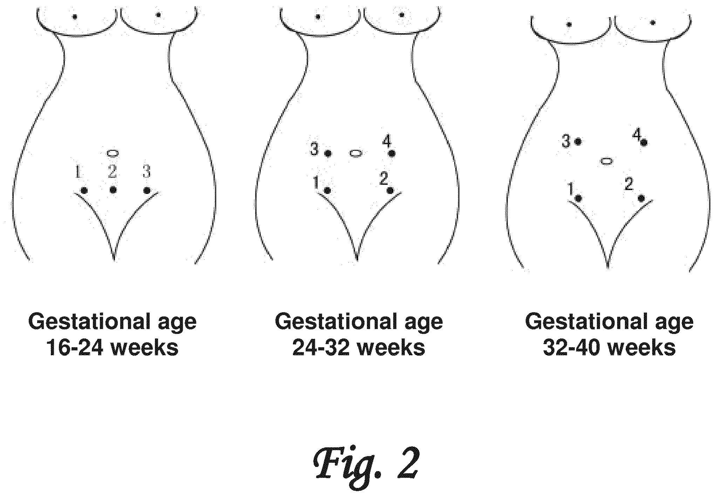

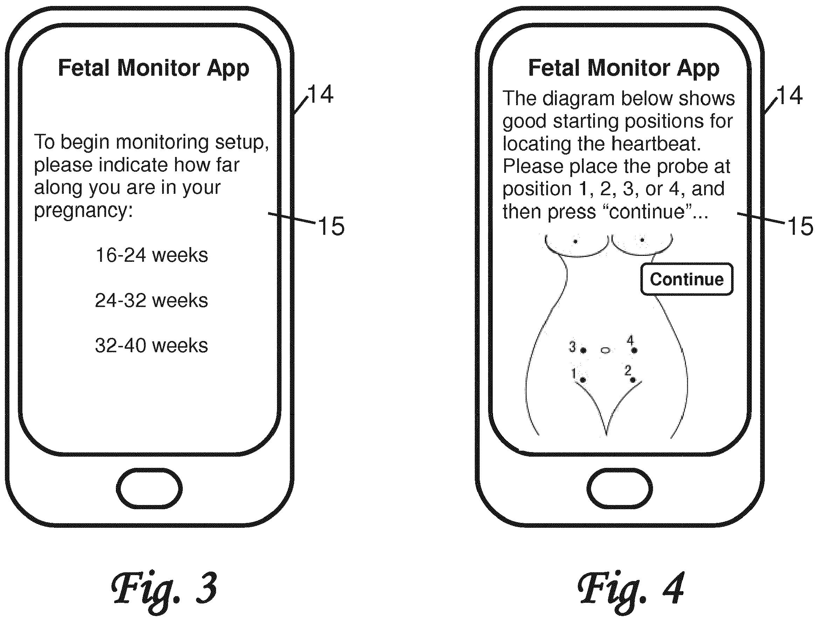

With continuing reference to Fig. 1 and with further reference to 2-4, before starting the placement assistance method (or, viewed alternatively, as a first step of the placement assistance method), it is advantageous to provide some guidance to the user (e.g. the mother, or another lay person assisting the mother) to initially place the ultrasound probe 10 on the mother's abdomen in the general vicinity of the womb. Because the womb shifts and enlarges over the course of a pregnancy, the likely good starting locations for placement of the ultrasound probe 10 depends upon gestational age of the fetus. Fig. 2 illustrates abdominal diagrams (e.g. suitably stored in a non-transitory storage medium of, or accessible by, the mobile device 14) depicting likely good locations for placing the ultrasound probe for three different gestational age ranges: 16-24 weeks (left diagram); 24-32 weeks (middle diagram); and 32-40 weeks (right diagram). In view of this, a suitable implementation of an initial placement user dialog 32 (Fig. 1) is shown in Figs. 3 and 4. In Fig. 3, the mobile device 14 displays a user dialog on its display 15 which queries the user to input the gestational age of the fetus. Preferably this user dialog (and all user dialogs of the placement assistance) employs terminology commonly used in discussing pregnancy with laypersons, e.g. in Fig. 3 the query is posed as: "please indicate how far along you are in your pregnancy". In the illustrative example, the user selected the time interval "24-32 weeks" which brings up the display shown in Fig. 4, which presents the middle diagram of Fig. 2 (appropriate for gestational age 24-32 weeks) along with the explanatory text: "The diagram below shows good starting positions for locating the heartbeat. Please place the probe at position 1, 2, 3, or 4, and then press 'continue'...." If the user had instead selected "16-24 weeks" at the dialog at Fig. 3, then the left diagram of Fig. 2 would be presented, and likewise if the user had selected "32-40 weeks" then the right diagram of Fig. 2 would be presented. When the user has placed the probe at the desired starting position, the user selects the "Continue" button in the display of Fig. 4 (e.g. using touchscreen input capability of the display 15).

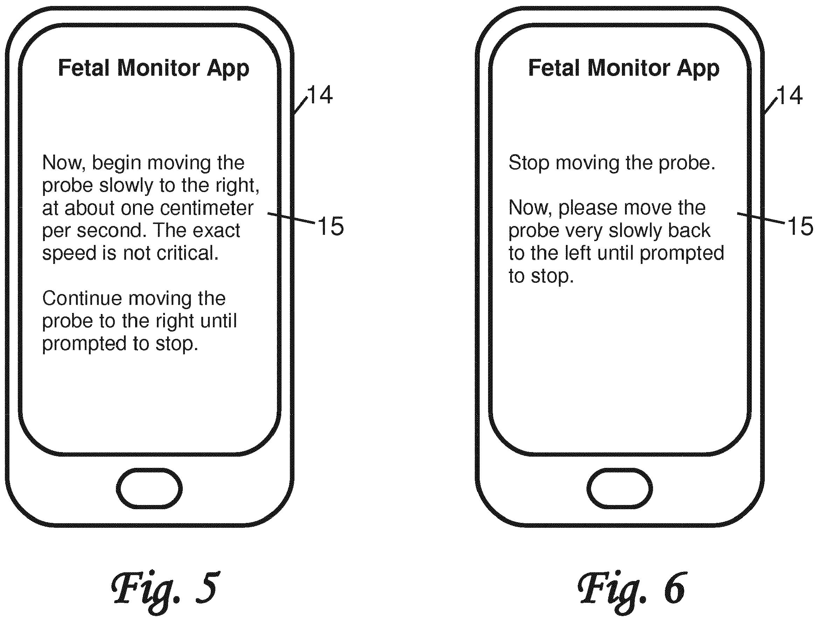

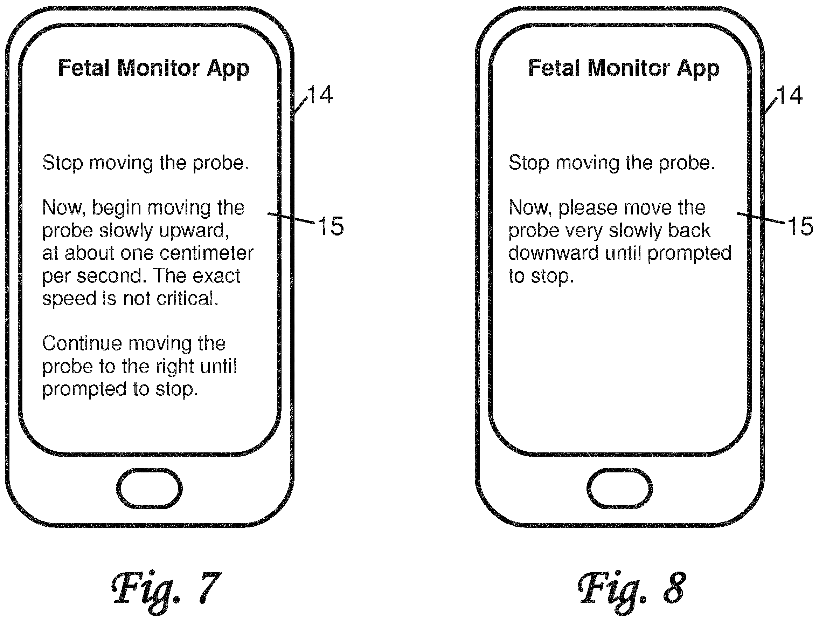

With continuing reference to Fig. 1 and with further reference to Figs. 5-7, the placement assistance method starts (or, alternatively, continues) with a user dialog display operation 34, a suitable embodiment of which is shown in Fig. 5, in which human-perceptible guidance is presented to move the Doppler ultrasound probe along a trajectory. In the illustrative example of Fig. 5, this presentation is by way of display of text stating: "Now, begin moving the probe slowly to the right, at about one centimeter per second. The exact speed is not critical. Continue moving the probe to the right until prompted to stop." Additionally or alternatively, the human-perceptible guidance may be presented by way of a voice recording or machine-generated speech. (For example, such an option maybe provided to assist a blind user). The illustrative suggested speed of one centimeter per second is an example, and the optimal speed depends on factors such as the lengths of the time bins (or other temporal resolution) of the signal strength versus time 30. After presenting the human-perceptible guidance per operation 34 and the illustrative example of Fig. 5, in an operation 36 the signal strength versus time 30 is monitored to detect a peak signal strength. It is assumed that the user is indeed following the guidance provided at 34 and is moving the ultrasound probe 10 slowly along the specified trajectory (to the right, i.e. along the lateral direction, in the example of Fig. 5) - accordingly, the signal strength versus time 30 should change over time as the probe approaches its closest position along the trajectory to the fetal heart. When it passes this closest point then the signal strength versus time 30 should peak and then start decreasing. The operation 36 detects the peak, e.g. by detecting the signal strength versus time 30 passing a maximum value which exceeds a first threshold T1 and then decreases. The first threshold T1 is chosen to avoid detecting noise peaks or other spurious signal fluctuations. In response to detecting the signal strength versus time 30 passing the maximum value in the operation 36, further human-perceptible guidance is provided at 38 to reverse the movement of the Doppler ultrasound probe along the trajectory and then presenting human-perceptible guidance to stop the movement of the Doppler ultrasound probe upon the signal strength versus time again attaining the maximum value. This is illustrated in the examples of Figs. 6 and 7, where in Fig. 6 the text is displayed: "Stop moving the probe. Now, please move the probe very slowly back to the left until prompted to stop." Assuming the user follows this guidance, it is expected that the signal strength versus time 30 will start to increase again until it reaches the maximum value initially detected at operation 36. At this point the further guidance: "Stop moving the probe" is displayed as shown in Fig. 7.

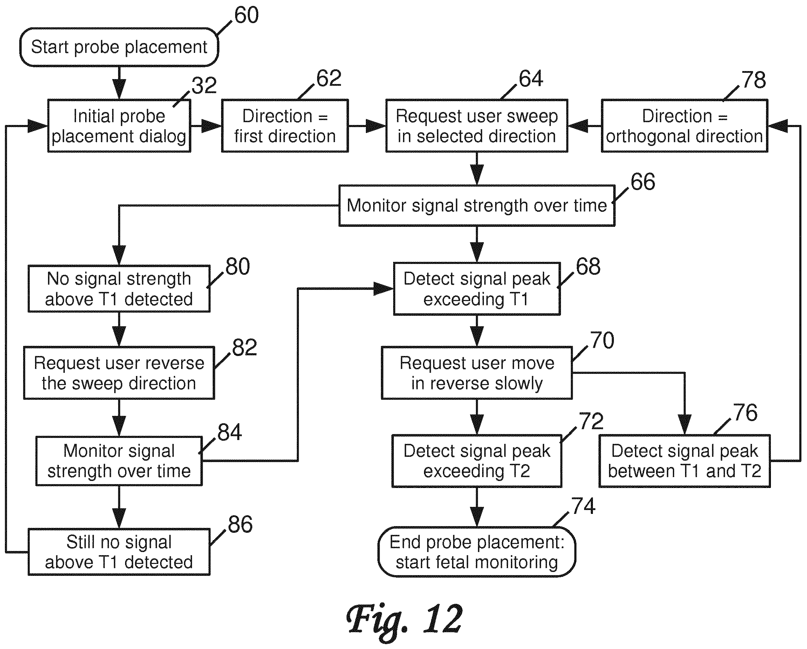

The guidance operations 34, 36, 38 of Fig. 1, corresponding to the user guidance displays shown in the examples of Figs. 5-7, effectively guide the user in performing a horizontal sweep of the probe starting from a position recommended at dialog 32 (see also Figs. 3 and 4). The rightward sweep assumes that the fetal heart is located to the right of the starting position recommended at dialog 32. This may usually be the case, since the recommended starting positions may be chosen to be typically to the left of the fetal heart. However, in some instances the starting point may be to the right of the fetal heart. In this case, sweeping to the right will not detect a peak. This can be handled by further dialogs to reverse direction, i.e. sweep to the left (not shown in Fig. 1, but see the example of Fig. 12).

As a further note, due to variations in the manual sweeping and other factors, it is understood that maximum of the signal strength versus time 30 that is detected at operation 36 maybe slightly different from the maximum value found at operation 38. Thus, during the reverse movement at 38 the maximum value is attained if it is within some error bar of the maximum value observed during the initial monitoring 36. An appropriate error bar depends on factors such as the noise level of the ultrasound probe 10 and fetal monitor 12, steadiness of a typical user in performing the manual probe sweeps, and so forth, and can be determined empirically from statistics on the peak variations and hard coded or included as a user-adjustable parameter.

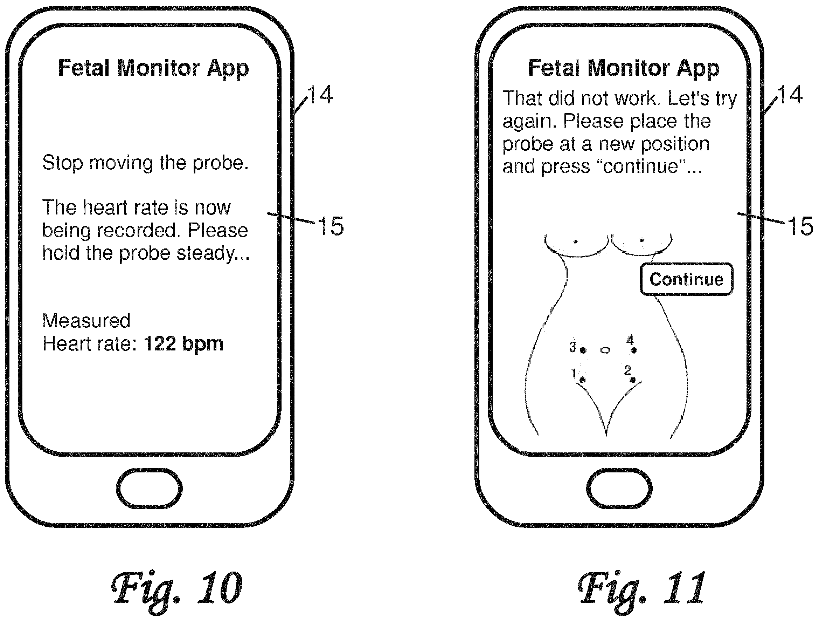

With continuing reference to Fig. 1 and further reference to Figs. 7-10, the operations 34, 36, 38 perform a sweep in the lateral anatomical direction, e.g. a linear horizontal sweep to the right, to locate the fetal heart. However, the fetal heart could be above or below (i.e. anatomically superior of, or inferior of) the horizontal line of this sweep. Hence, the guidance operations 34, 36, 38 are repeated as guidance operations 40, 42, 44 along a different trajectory, e.g. a vertical upward or downward (i.e., anatomically, superior or inferior) linear trajectory that is orthogonal to the first (e.g. lateral) trajectory. Hence, the placement assistance method continues with a user dialog display operation 40, a suitable embodiment of which is shown in Fig. 7, in which human-perceptible guidance is presented to move the Doppler ultrasound probe along a different trajectory. In the illustrative example of Fig. 7, this presentation is byway of display of text stating: "Now, begin moving the probe slowly upward, at about one centimeter per second. The exact speed is not critical. Continue moving the probe to the right until prompted to stop." Again, this (and all other human-perceptible guidance) could additionally or alternatively be in some other form, such as presented by way of a voice recording or machine-generated speech. After presenting the human-perceptible guidance per operation 40 and the illustrative example of Fig. 7, in an operation 42 the signal strength versus time 30 is again monitored to detect a peak signal strength. It is assumed that the user is indeed following the guidance provided at 40 and is moving the ultrasound probe 10 slowly along the specified trajectory (upward, i.e. along the superior direction, in the example of Fig. 7) - accordingly, the signal strength versus time 30 should change over time as the probe approaches its closest position along the trajectory to the fetal heart. When it passes this closest point then the signal strength versus time 30 should peak and then start decreasing. The operation 42 detects the peak, e.g. by detecting the signal strength versus time 30 passing a maximum value which exceeds the first threshold T1 and then decreases. (Note that the maximum value here is likely to be larger than the maximum value detected in the first horizontal sweep monitoring operation 36). In response to detecting the signal strength versus time 30 passing the maximum value in the operation 42, further human-perceptible guidance is provided at 44 to reverse the movement of the Doppler ultrasound probe along the trajectory and then presenting human-perceptible guidance to stop the movement of the Doppler ultrasound probe upon the signal strength versus time again attaining the maximum value. This is illustrated in the examples of Figs. 8 and 10, where in Fig. 8 the text is displayed: "Stop moving the probe. Now, please move the probe very slowly back to the left until prompted to stop." Assuming the user follows this guidance, it is expected that the signal strength versus time 30 will start to increase again until it reaches the maximum value initially detected at operation 42. At this point the further guidance: "Stop moving the probe" is displayed as shown in Fig. 10.

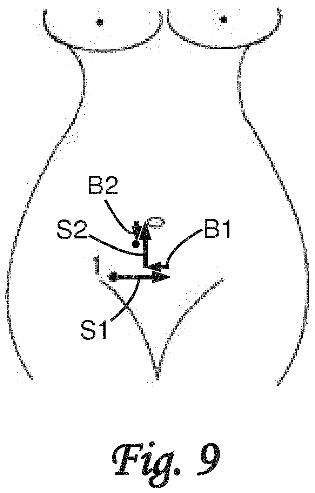

As diagrammatically shown in Fig. 9, the result of the sequence of guidance operations 34, 36, 38, 40, 42, 44 is to guide the user in sweeping the ultrasound probe 10 along a path that starts from illustrative starting position "1"; then moves to the right along path S1 (operations 34, 36 and Figs. 5 and 6); then goes back along reverse path B1 to center on the signal strength peak (operation 38 and Figs. 6 and 7); then upward along path S2 (operations 40, 42 and Figs. 7 and 8); and finally back along reverse path B2 to center on the signal strength peak (operation 44 and Figs. 8 and 10).

In principle, the foregoing should be sufficient to position the ultrasound probe 10 optimally with respect to the fetal heart. However, in practice some error may still be present, e.g. due to distortions in the ultrasound reflections or so forth, or by providing guidance to sweep in a wrong direction in one of the guidance operations 34, 40. To check for this, in a decision operation 46 it is determined whether the maximum value of the signal strength versus time 30 which is reached at the end of the operation 44 exceeds a second threshold T2, which is greater than the threshold T1. The threshold T2 is selected as a threshold which, exceeded, ensures that a sufficiently strong ultrasound signal indicative of fetal heartbeat is being detected in order to perform accurate fetal monitoring. Again, the threshold T2 depends on numerous factors such as the sensitivity of the fetal monitor 12, the type(s) of fetal monitoring to be performed, and/or so forth, and hence the second threshold T2 is preferably chosen empirically.

With continuing reference to Fig. 1 and further reference to Fig. 10, if at decision operation 46 it is determined that the signal strength is sufficient (i.e. exceeds second threshold T2) then fetal monitoring is started at operation 48 of Fig. 1. A suitable notification is preferably displayed, e.g. as shown in the example of Fig. 10 which provides the human perceptible (e.g. displayed) guidance: "The heart rate is now being recorded. Please hold the probe steady...." Optionally, as shown in Fig. 10 the measured fetal heart rate may be displayed. The fetal monitoring may optionally include monitoring other aspects of the fetus, such as counting fetal movements, analyzing the fetal heartbeat pattern and/or the shape of the fetal heartbeats to detect any abnormalities that may be so detected, and/or so forth.

With continuing reference to Fig. 1 and further reference to Fig. 11, if on the other hand at decision operation 46 it is determined that the signal strength is not sufficient (i.e. does not exceed second threshold T2) then flow may return to guidance operation 34 to attempt a second pass at a horizontal sweep. If the signal strength is too low, then the process may need to be re-started, e.g. using a further user guidance dialog such as the example provided in Fig. 11, in which the user is informed: "That did not work. Let's try again. Please place the probe at a new position and press "continue." This amounts to flow returning to the initial probe placement dialog 32 but with the guidance text revised slightly to reflect the fact that this is a second (or more) attempt at probe placement.

In general, the disclosed approach entails monitoring signal strength versus time 30 of a Doppler ultrasound signal measured by a Doppler ultrasound probe 10 and, during the monitoring, performing a probe placement assistance method that includes presenting human-perceptible guidance to move the Doppler ultrasound probe along a trajectory and then detecting a peak in the signal strength versus time which exceeds the first threshold (T1) and then presenting human-perceptible guidance to stop movement of the Doppler ultrasound probe. This may be repeated one or more times for different trajectories, such as the horizontal and vertical trajectories (or, anatomically, lateral and superior/inferior trajectories) of the foregoing example. Various further guidance can be provided, such as going back to center on the peak as per guidance operations 38, 44. Other variants in the provided guidance can be provided, as in the next example which is presented in the form of a probe placement guidance method suitably performed by the system of Fig. 1.

With reference now to Fig. 12, in this variant example the method starts at 60, with the initial probe placement dialog 32 already described with reference to Figs. 1-4. Thereafter, for a first sweep direction 62 (e.g., horizontally to the right), in an operation 64 human-perceptible guidance is presented (e.g. displayed, and/or presented by recorded or synthesized audio speech) to request the user move the probe in the requested direction (initially the first direction 62). In an operation 66, signal strength versus time is monitored while the user is assumed to be moving the probe in the requested direction. If a signal peak is detected at 68 that exceeds the first threshold T1 (e.g. by detecting the monitored signal strength reaches a maximum and then begins to decrease), then at 70 human-perceptible guidance is presented to ask the user to move in reverse slowly until the signal strength peak is again reached. At this point, if at 72 it is determined that the peak signal strength exceeds the second threshold T2 then as indicated at 74 the signal strength is deemed sufficient for fetal monitoring and the fetal monitoring is started. On the other hand, if at 76 it is determined that the peak signal strength exceeds first threshold T1 (as it should since a signal exceeding threshold T1 was detected at 68) but does not exceed second threshold T2, then a different, e.g. orthogonal direction 78 is selected (e.g., upward), and process flow returns to guidance operation 64 in order to present human-perceptible guidance to request the user move the probe in the requested direction (now the orthogonal direction 78). This loop 64, 66, 68, 70, 76, 78 can be repeated multiple times if needed, until at 72 a peak signal strength exceeding the second threshold T2 is detected.

Fig. 12 also depicts a process for remediating the situation in which a sweep is in the wrong direction. If the monitoring operation 66 continues for some duration of time without detecting a signal above the first threshold T1, this is detected at 80. The first threshold T1 is chosen to filter out signals that are at or near the noise level - if the sweep is in the wrong direction then the signal will generally be below first threshold T1. In response to the detection 80 of no meaningful signal strength, further human-perceptible guidance is presented at 82 asking the user to reverse direction of the sweep (e.g., if the user was sweeping to the right then the request would be to sweep to the left; or, if the user was sweeping upward then the request would be to sweep downward). At 84 the signal strength over time is again monitored. If a signal strength peak is detected that exceeds the first threshold T1 at 68, then flow returns to the main loop already described. On the other hand, if at 86 the further monitoring 84 still does not detect a signal strength exceeding the first threshold T1 (e.g. after the same duration used in the detection 80), then flow returns to block 32 to ask that the user restart the process with a new starting placement, e.g. as described previously with reference to Fig. 11.

In the foregoing examples, the user is requested to perform a sweep along a linear trajectory, e.g. to the right, or upward, or so forth, and a two-dimensional area of the abdomen is probed by sweeping along a first linear trajectory followed by sweeping along a second, orthogonal linear trajectory. This provides a systematic approach for optimizing over the area of the abdomen. However, other approaches are contemplated. For example, in one approach, the user may be first asked to position the probe at the center of the abdomen and then human-perceptible guidance may be presented to move the Doppler ultrasound probe 10 along an expanding spiral trajectory, and to detect a peak in the signal strength versus time 30 which exceeds the second threshold T2 and then presenting human-perceptible guidance to stop movement of the Doppler ultrasound probe. This approach presumes that the expanding spiral trajectory is likely to pass over the optimal location proximate to the fetal heart. In one contemplated approach, the spiral trajectory is attempted first as it is efficient, and if it fails then orthogonal linear trajectories are sampled as described previously.

The invention has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims or the equivalents thereof.

## Claims

1. A probe placement assistance method for fetal monitoring, the probe placement assistance method comprising:
monitoring signal strength versus time (30) of a Doppler ultrasound signal (18) measured by a Doppler ultrasound probe (10); and,
during the monitoring, presenting human-perceptible guidance to move the Doppler ultrasound probe along a trajectory and then detecting a peak in the signal strength versus time which exceeds a threshold (T1, T2) and then presenting human-perceptible guidance to stop movement of the Doppler ultrasound probe.

2. The probe placement assistance method of claim 1 wherein the monitoring includes:
determining a signal strength value of the Doppler ultrasound signal (18) for each time bin of a time sequence of time bins, the signal strength versus time (30) being the sequence of signal strength values for the time sequence of time bins.

3. The probe placement assistance method of claim 2 wherein the signal strength value of the Doppler ultrasound signal (18) for each time bin is determined as one of:
proportional to a sum of squared samples of a signal envelope determined by rectifying the Doppler ultrasound signal and low-pass filtering the rectified Doppler ultrasound signal; or
proportional to a maximum value of the Doppler ultrasound signal over the time bin.

4. The probe placement assistance method of any one of claims 1-3 wherein detecting the peak in the signal strength versus time includes:
detecting the signal strength versus time (30) passing a maximum value which exceeds a first threshold (T1) and then decreases; and
in response to detecting the signal strength versus time passing the maximum value, presenting human-perceptible guidance to reverse the movement of the Doppler ultrasound probe along the trajectory and then presenting human-perceptible guidance to stop the movement of the Doppler ultrasound probe upon the signal strength versus time again attaining the maximum value.

5. The probe placement assistance method of any one of claims 1-4 further comprising:
conditional upon the peak in the signal strength versus time (30) exceeding a first threshold (T1) but not exceeding a second threshold (T2) that is higher than the first threshold, repeating the presenting and then detecting and then presenting human-perceptible guidance to stop movement for a different trajectory; and
conditional upon the peak in the signal strength versus time exceeding the second threshold, causing a fetal monitor (12) operatively connected with the Doppler ultrasound probe (10) to perform fetal monitoring using the Doppler ultrasound probe including at least monitoring a fetal heart rate.

6. The probe placement assistance method of claim 5 wherein the trajectory is a first linear trajectory, the different trajectory is a second linear trajectory, and the first linear trajectory and the second linear trajectory are orthogonal to each other.

7. The probe placement assistance method of any one of claims 1-6 further comprising, during the monitoring and prior to presenting human-perceptible guidance to move the Doppler ultrasound, causing a display (15) to present an abdominal diagram with one or more suggested initial placement positions for the Doppler ultrasound probe (10) indicated on the abdominal diagram.

8. The probe placement assistance method of any one of claims 1-7 wherein the human-perceptible guidance is presented by at least one of:
displaying the human-perceptible guidance on a display (15) operatively connected with the electronic processor, and
outputting the human-perceptible guidance audibly via a loudspeaker operatively connected with the electronic processor.

9. A fetal monitoring device comprising:
a fetal monitor (12);
a Doppler ultrasound probe (10) operatively connected with the fetal monitor;
one or more electronic processors each integral with or operatively connected with the fetal monitor; and
a non-transitory storage medium storing instructions which are readable and executable by the one or more electronic processors to perform a fetal monitoring method including:
monitoring signal strength versus time (30) of a Doppler ultrasound signal (18) measured by the Doppler ultrasound probe; and,
during the monitoring, performing at least one iteration of a probe placement assistance method comprising presenting human-perceptible guidance to move the Doppler ultrasound probe along a trajectory and then detecting a peak in the signal strength versus time which exceeds a threshold (T1, T2) and then presenting human-perceptible guidance to stop movement of the Doppler ultrasound probe; and
after completion of a last iteration of the probe placement assistance method, performing fetal monitoring using the Doppler ultrasound probe including at least monitoring a fetal heart rate.

10. The fetal monitoring device of claim 9 wherein the monitoring of the signal strength versus time (30) of the Doppler ultrasound signal (18) includes:
determining a signal strength value of the Doppler ultrasound signal (18) for each time bin of a time sequence of time bins, the signal strength versus time (30) being the sequence of signal strength values for the time sequence of time bins.

11. The fetal monitoring device of any one of claims 9-10 wherein detecting the peak in the signal strength versus time includes:
detecting the signal strength versus time (30) passing a maximum value which exceeds a first threshold (T1) and then decreases; and
in response to detecting the signal strength versus time passing the maximum value, presenting human-perceptible guidance to reverse the movement of the Doppler ultrasound probe along the trajectory and then presenting human-perceptible guidance to stop the movement of the Doppler ultrasound probe upon the signal strength versus time again attaining the maximum value.

12. The fetal monitoring device of any one of claims 9-11 wherein the fetal monitoring method includes performing at least first and second iterations of the probe placement assistance method wherein the first iteration includes presenting human-perceptible guidance to move the Doppler ultrasound probe along a first linear trajectory and the second iteration includes presenting human-perceptible guidance to move the Doppler ultrasound probe along a second linear trajectory that is orthogonal to the first linear trajectory.

13. The fetal monitoring device of any one of claims 9-12 further comprising:
a display (15);
wherein the fetal monitoring method further comprises, prior to performing a first iteration of the probe placement assistance method, causing the display to present an abdominal diagram with one or more suggested initial placement positions for the Doppler ultrasound probe (10) indicated on the abdominal diagram.

14. The fetal monitoring device of any one of claims 9-13 wherein the one or more electronic processors include an electronic processor that is integral with the fetal monitor (12) and a cellular telephone or tablet computer (14) that is in wireless communication with the fetal monitor.

15. A non-transitory computer readable medium having stored thereon program code readable and executable by one or more electronic processors to perform operations including:
monitoring signal strength versus time (30) of a Doppler ultrasound signal (18) measured by a Doppler ultrasound probe (10); and,
during the monitoring, performing a probe placement assistance method including presenting human-perceptible guidance to move the Doppler ultrasound probe along a trajectory and then detecting a peak in the signal strength versus time which exceeds a threshold (T1, T2) and then presenting human-perceptible guidance to stop movement of the Doppler ultrasound probe.
